# EUROPEAN PATENT SPECIFICATION

(11) **EP 2 464 285 B1**
(45) Date of publication and mention of the grant of the patent: **25.09.2013**
(21) Application number: 11754911.3
(22) Date of filing: 26.08.2011
(51) Int. Cl.: A61B 5/0476, G06F 19/00, A61N 1/00

(54) **A MONITORING OR PREDICTING SYSTEM AND METHOD OF MONITORING OR PREDICTING**
ÜBERWACHUNGS- ODER VORHERSAGESYSTEM SOWIE ÜBERWACHUNGS- ODER VORHERSAGEVERFAHREN
SYSTEME DE SURVEILLANCE OU DE PREDICTION ET PROCEDE DE SURVEILLANCE OU DE PREDICTION

(30) Priority: 27.08.2010 GB 201014333
(43) Date of publication of application: 20.06.2012
(73) Proprietor: Neuropro Limited, Road Town, Tortola (VG)
(72) Inventor: Juffali, Walid, Jeddah 21431 (SA); El-Imad, Jamil, 8001 Zürich (CH)
(74) Representative: Hoarton, Lloyd Douglas Charles
(86) International application number: PCT/GB2011/051616
(87) International publication number: WO 2012/025765

(56) References cited:
- WO-A2-2010/115939
- US-A- 5 995 868
- US-A1- 2006 111 644
- US-B1- 6 658 287
- WALID JUFFALI ET AL: "The WiNAM project: Neural data analysis with applications to epilespy", BIOMEDICAL CIRCUITS AND SYSTEMS CONFERENCE (BIOCAS), 2010 IEEE, IEEE, 3 November 2010 (2010-11-03), pages 45-48, XP031899695, DOI: 10.1109/BIOCAS.2010.5709567 ISBN: 978-1-4244-7269-7

## Description

### Description of Invention

### Field of the invention

This invention relates to a monitoring or predicting system and to a method of monitoring or predicting neurological electrical signals.

### Background

Major attempts are constantly being made to monitor and predict epileptic seizures. Most predictive methods analyse electrical signals representing neuronal activity in the brain using electrode pickups located at the level of the scalp, externally, under the scalp or deep within the brain.

An electroencephalogram (EEG) is a system for recording electrical activity in the brain produced by the firing of neurons within the brain. Multiple electrodes are placed around the scalp but electrodes can also be placed in direct contact with the brain or within the brain. The EEG signal is composed of different wave patterns operating in a spectrum going from below 4Hz to over 100Hz. There are other mechanisms for detecting and recording neuronal activity such as an electrocorticogram (ECoG) where the signal is derived directly from the cerebral cortex or functional magnetic resonance imaging (FMRI).

Epilepsy is just one example of a potential neurological episode.

WALID JUFFALI et.al: "The WiNAM project: Neural data analysis with applications to epilepsy" BIOMEDICAL CIRCUITS AND SYSTEMS CONFERENCE (BIOCAS), 2010 IEEE, IEEE 3 November 2010, pages 45-48, (XP031899695, D01: 10.1109/BIOCAS, 2010, 5709567, ISBN: 978-1-4244-7269-7) discloses a novel algorithmic method based on an ngram approach and applies it to ECoG and deep brain neural data for analysis of epileptic seizures.

US 6,658,287 discloses a method, and system for predicting the onset of a seizure prior to electrograph onset in an individual.

During an "off-line" mode, signals representing brain activity of an individual (either stored or real time) are collected, and features are extracted from those signals. A subset of features, which comprise a feature vector, are selected by a predetermined process to most efficiently predict (and detect) a seizure in that individual. An intelligent prediction subsystem is also trained "off-line" based on the feature vector derived from those signals.

During "on-line" operation, features are continuously extracted from real time brain activity signals to form a feature vector, and the feature vector is continuously analyzed with the intelligent prediction subsystem to predict seizure onset in a patient.

US 2006/111644 discloses methods and systems for patient-specific seizure onset detection.

In one embodiment, at least one EEG waveform of the patient is recorded, and at least one epoch (sample) of the waveform is extracted. The waveform sample is decomposed into one or more subband signals via a wavelet decomposition of the waveform sample, and one or more feature vectors are computed based on the subband signals. A seizure onset can then be identified based on classification of the feature vectors to a seizure or a non-seizure class by comparing the feature vectors with a decision measure previously computed for that patient. The decision measure can be derived based on reference seizure and non-seizure EEG waveforms of the patient.

US 2005/197590A discloses a system (10) which analyzes signals representative of a subject's brain activity in a signal processor (12) for information indicating the subject's current activity state and for predicting a change in the activity state.

One preferred embodiment uses a combination of nonlinear filtering methods to perform real-time analysis of the electro-encephalogram (EEG) or electrocorticogram (ECoG) signals from a subject patient for information indicative of or predictive of a seizure, and to complete the needed analysis at least before clinical seizure onset. The preferred system then performs an output task for prevention or abatement of the seizure, or for recording pertinent data.

WO 2010/115939 discloses a method for the real-time identification of seizures in an Electroencephalogram (EEG) signal.

The method provides for patient-independent seizure identification by use of a multi-patient trained generic Support Vector Machine (SVM) classifier. The SVM classifier is operates on a large feature vector combining features from a wide variety of signal processing and analysis techniques. The method operates sufficiently accurately to be suitable for use in a clinical environment. The method may also be combined with additional classifiers, such a Gaussian Mixture Model (GMM) classifier, for improved robustness, and one or more dynamic classifiers such as an SVM using sequential kernels for improved temporal analysis of the EEG signal.

### Brief summary of the invention

The present invention provides a monitoring or predicting system and method as claimed.

In order that the present invention may be more readily understood, embodiments thereof will now be described, by way of example, with reference to the accompanying drawings, in which:
Figure 1 shows a first set of potential electro positions for use with a device or system embodying the present invention;
Figure 2 shows another potential set of electro positions for use with a device or system embodying the present invention;
Figure 3 is a graph showing a relationship between seizure risk and neuronal activity signal anomalies;
Figure 4 is a block diagram of a monitoring or predicting device embodying the present invention;
Figure 5 is a schematic block diagram showing a system embodying the present invention for gather and analysing neuronal activity signals embodying the present invention;
Figure 6 is a table of anomaly results achieved using an embodiment of the system of figure 5;
Figure 7 shows an example of the pattern count during pre-itcal and ictal periods, the ictal period being shaded;
Figure 8 is a graph showing an example of the pattern count varying over time;
Figure 9 shows a graphical user interface designed to analyse neuronal activity data signals in accordance with an embodiment of the invention;

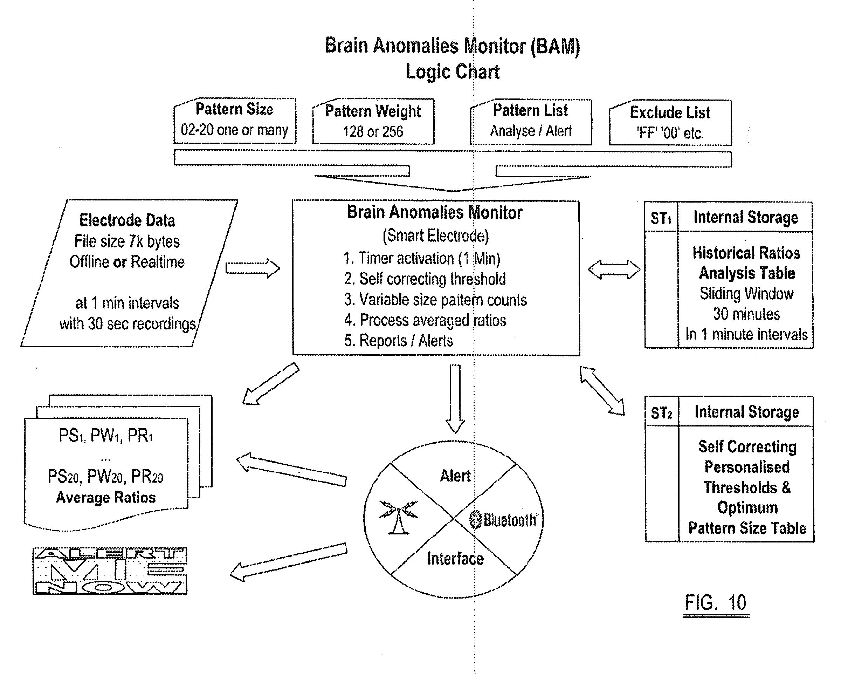

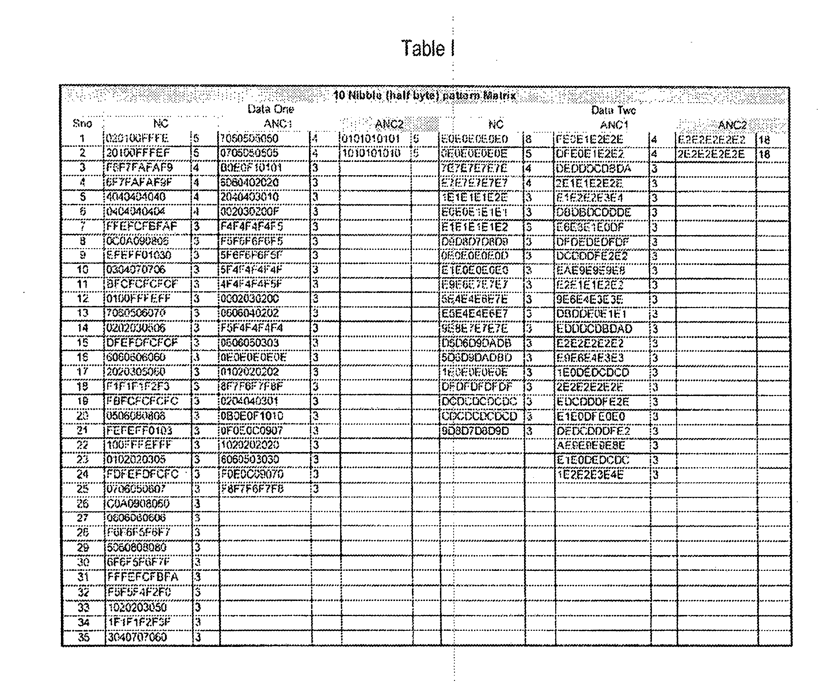

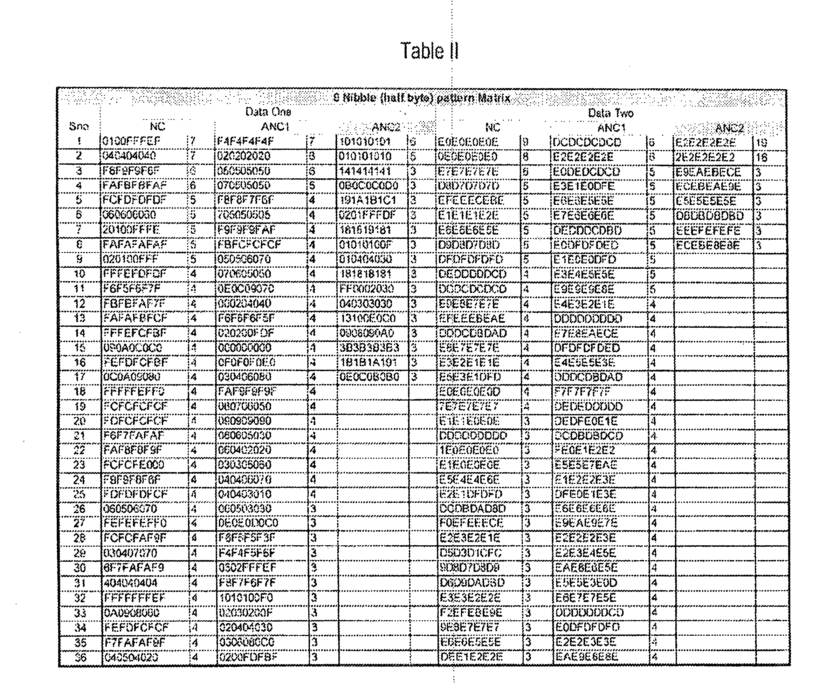

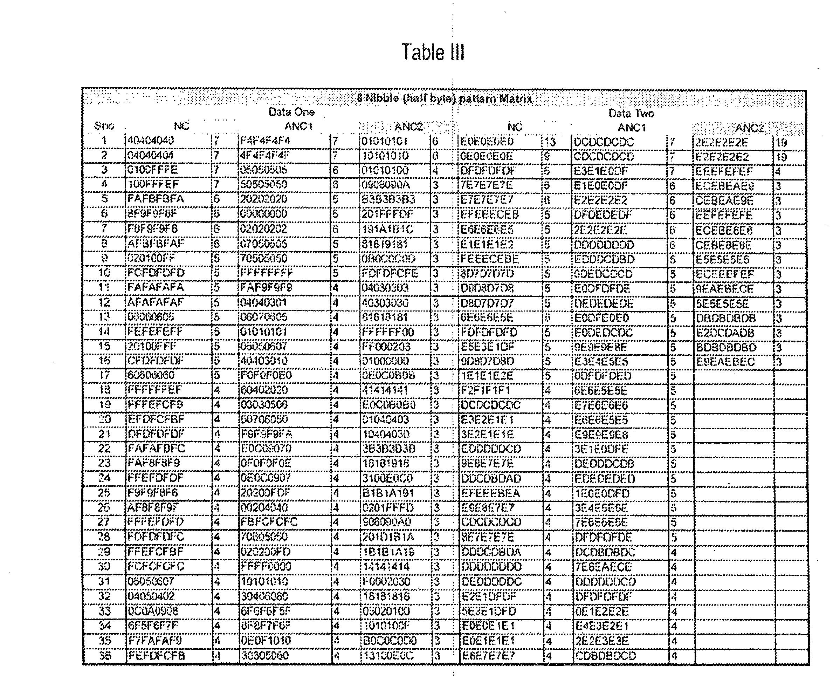

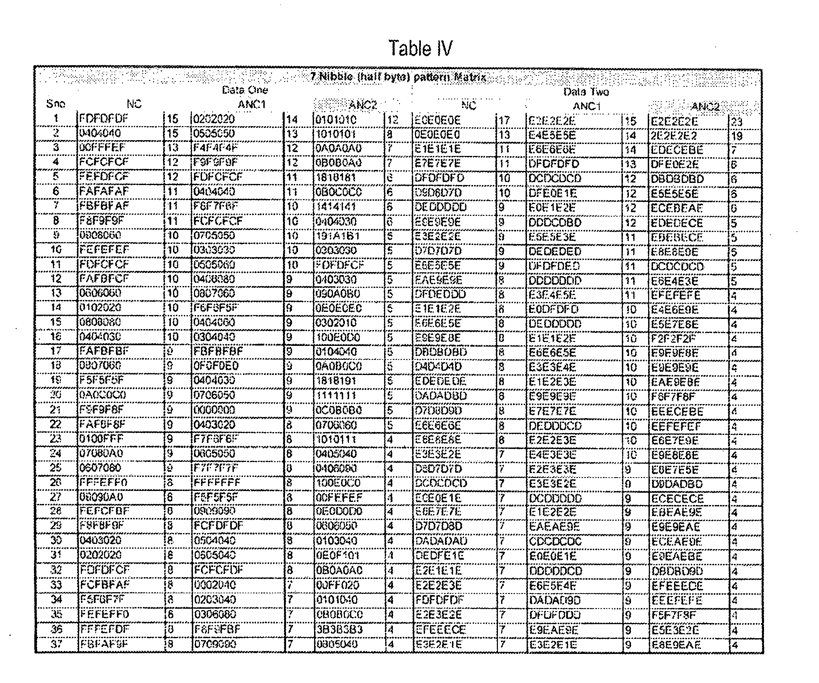

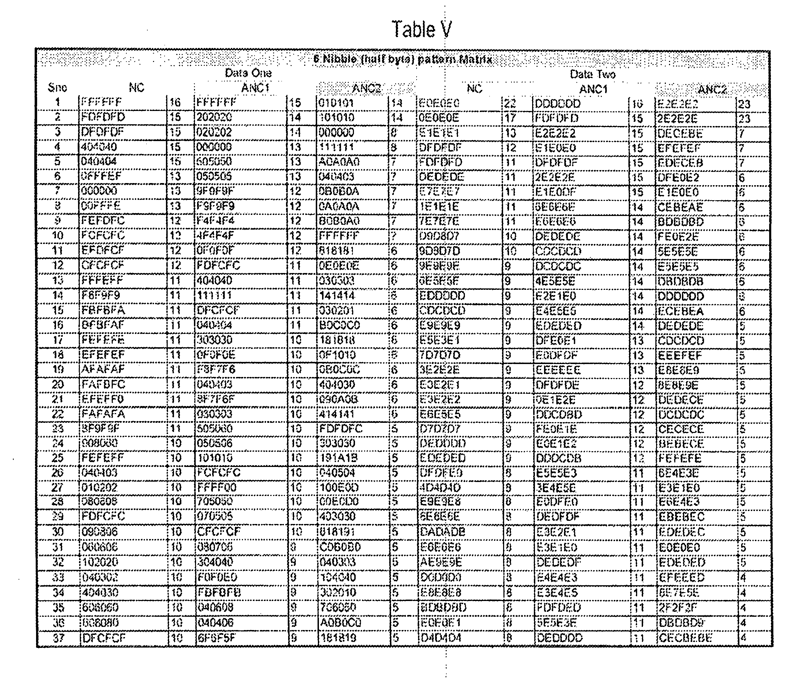

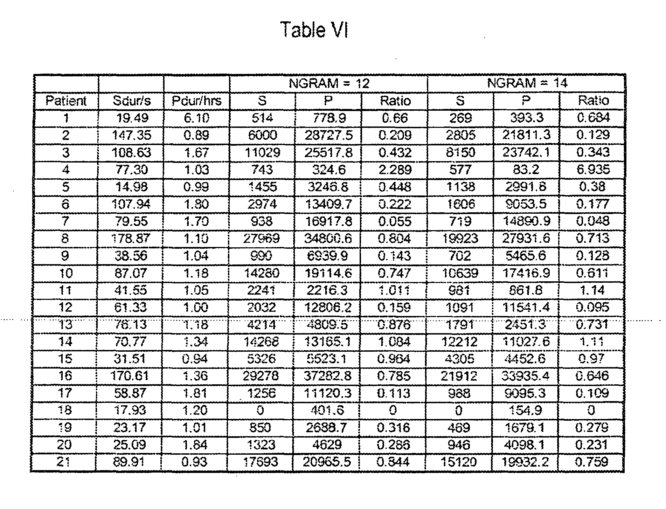

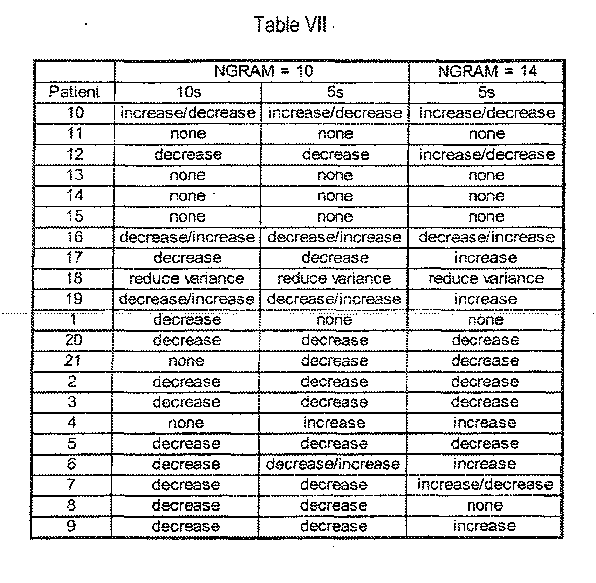

Figure 10 is another block diagram representation of a monitoring or predicting device embodying the present invention.
   Table I shows a 10 nibble pattern matrix;
   Table II shows an example of a first part of a 9 nibble pattern matrix;
   Table III shows an example of a first part of an 8 nibble pattern matrix;
   Table IV shows an example of a first part of a 7 nibble pattern matrix;
   Table V shows an example of a first part of a 6 nibble pattern matrix;
   Table VI shows some initial results distinguishing pre-ictal from ictal periods;
   Table VII shows historical results related to pattern count changes when analysing full data sets;

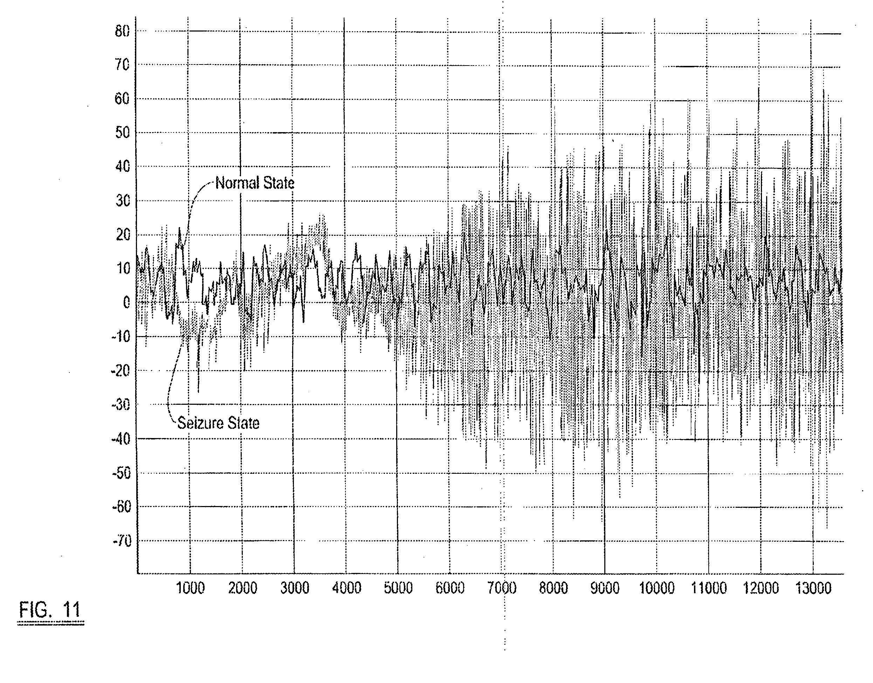

Figure 11 is a snap-shot of over 12000 electronic readings taken from a single patient, the bold trace reflecting normal state and the fainter trace representing readings taken from the same patient during seizure;

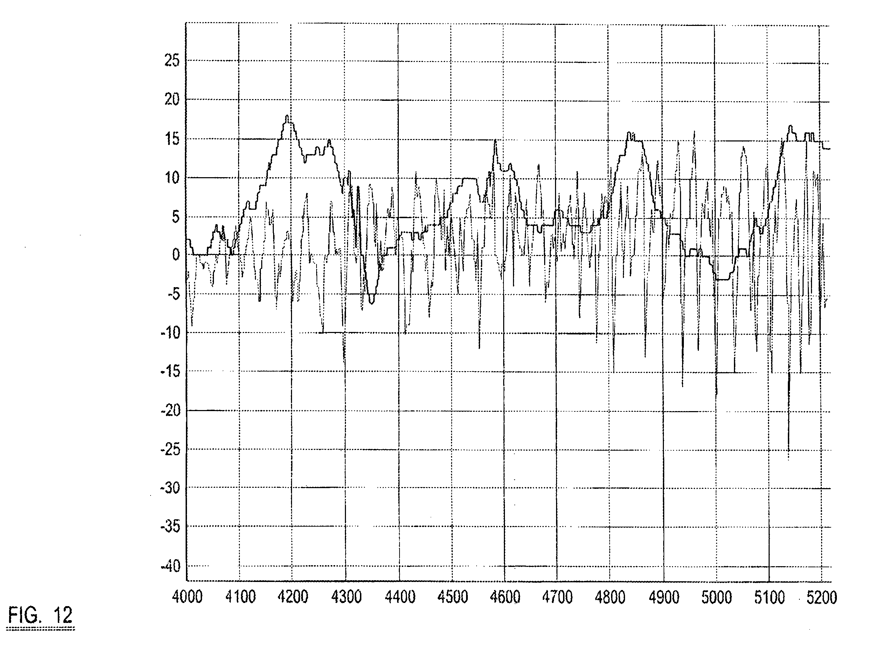

Figure 12 is a detail of the electronic readings from figure 11 running from electronic readings 4000 to 5200;

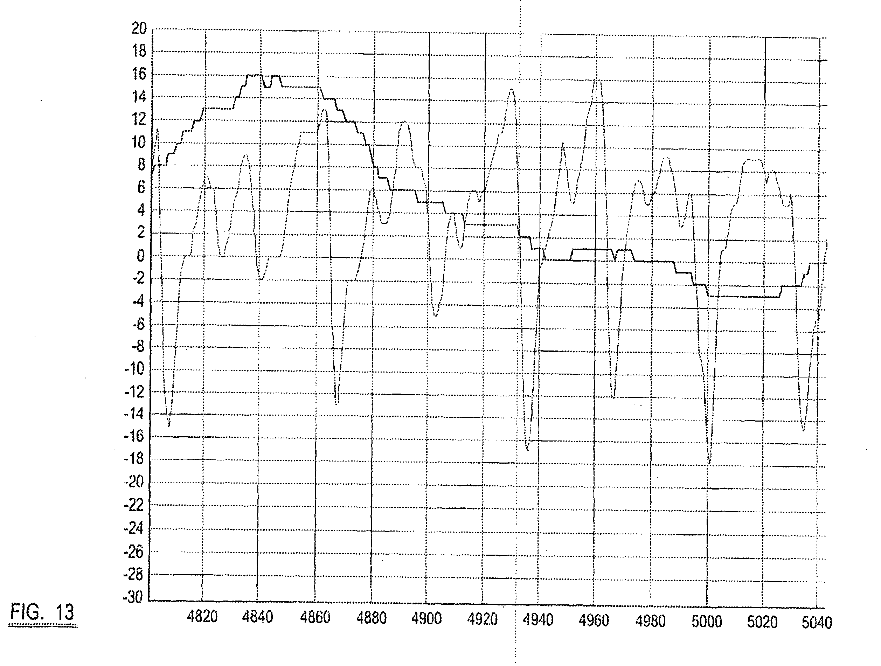

Figure 13 is a detail of the electronic readings from figure 11 running from electronic readings 4800 to 5040;

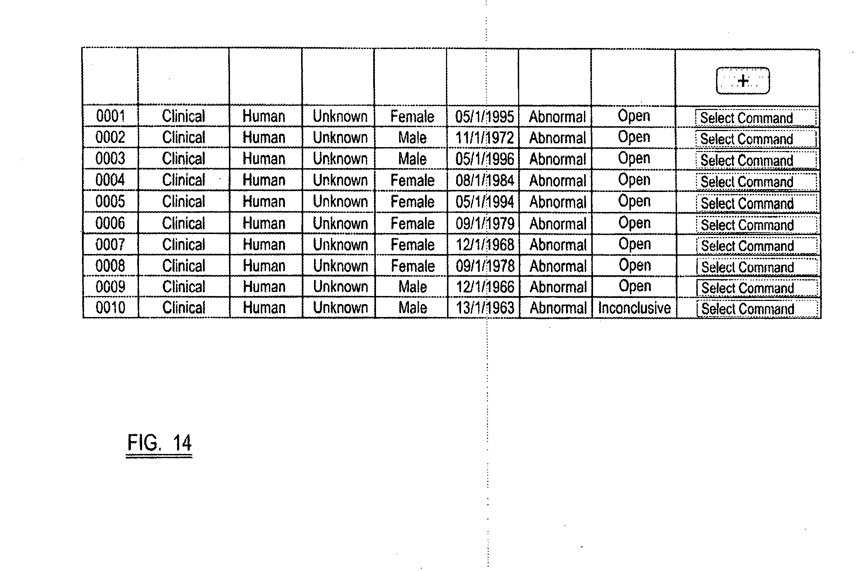

Figure 14 shows a case list of available data for different patients;

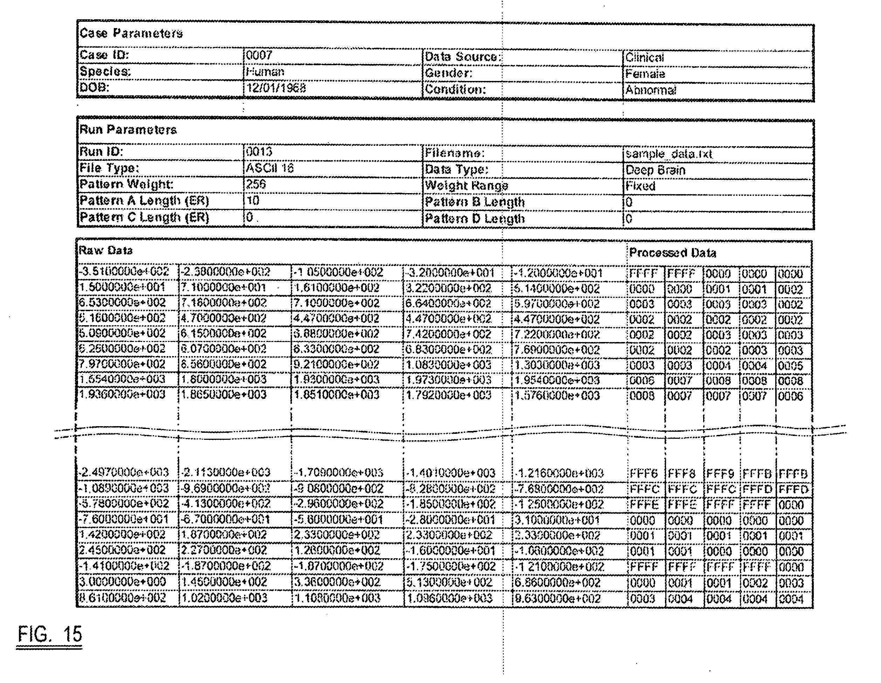

Figure 15 shows raw data and process data for case 7;

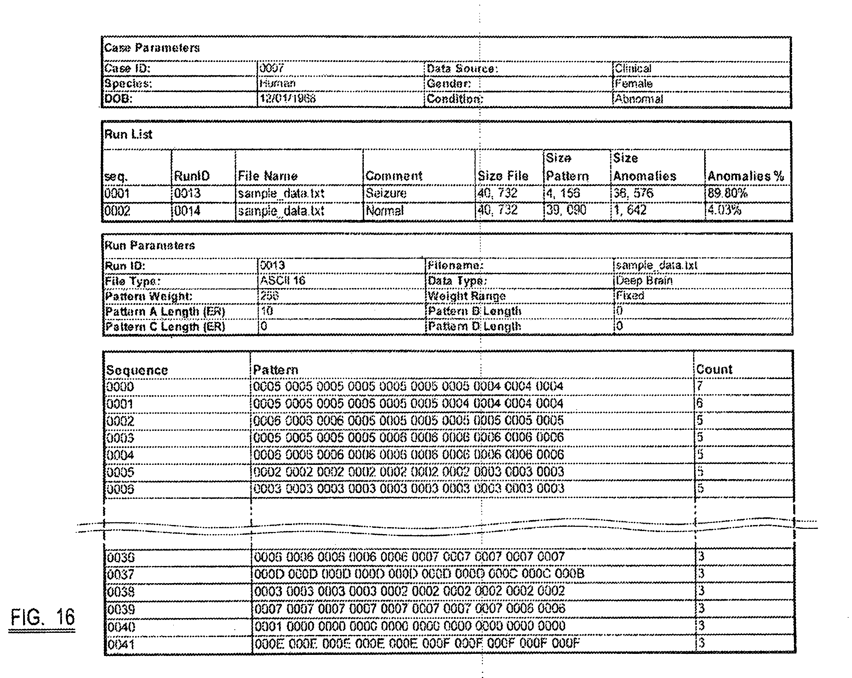

Figure 16 gives a pattern count for 10 nibble patterns identified during seizure conditions - run 13 and a summary of the anomaly percentage; and

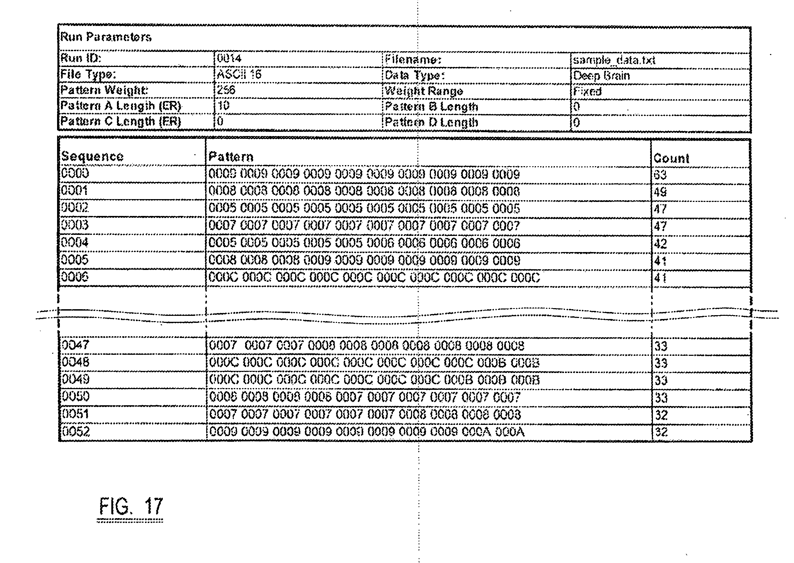

Figure 17 gives the same information as the pattern count shown in figure 16 but for run 14 taken from the same patient during a normal state.

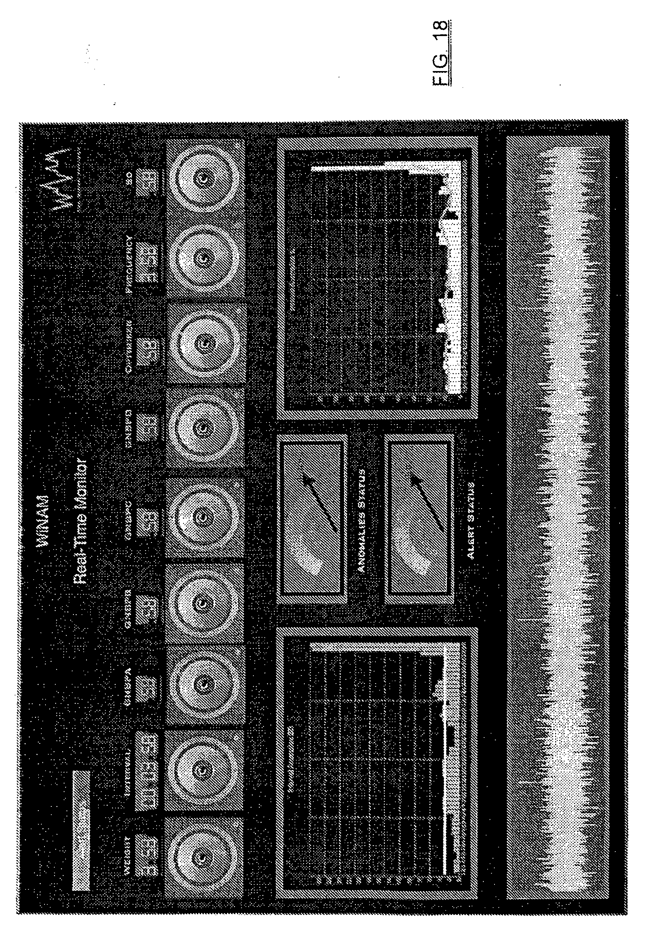

Figure 18 shows a second graphical user interface designed to analyse neuronal activity data signals in accordance with an embodiment of the invention.

One embodiment of the present invention is a neurological signal monitoring or predicting device. The device receives input from one or more sensors which are suitable for receiving signals indicative of neuronal activity from the brain.

Electrodes or electrical contacts are the preferable form of sensors to detect neuronal activity from the brain, i.e. neuronal activity sensors. For the sake of convenience, this specification refers to neuronal activity sensors as electrodes or electrical contacts but non-electrical sensors to detect or derive neuronal activity are possible alternatives or equivalents to electrical sensors. As well as being used as inputs, the electrical contacts may also be configured as outputs to provide neuronal stimulation to a part or parts of the brain.

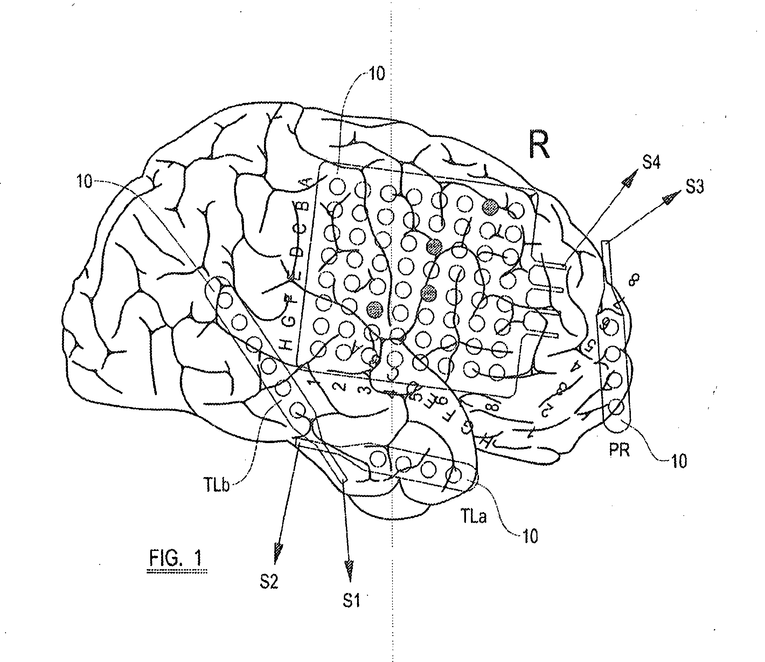

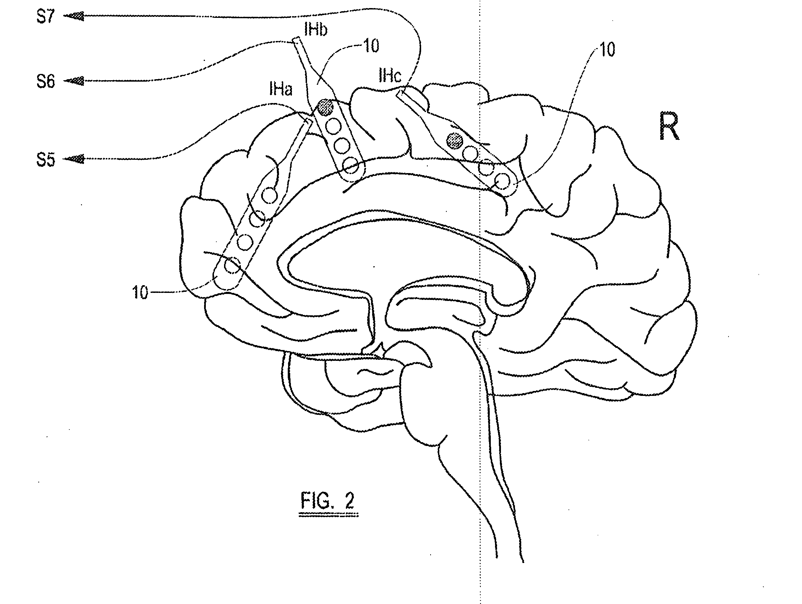

There are conventions for positioning and fixing of EEG electrodes (see figures 1 and 2) so that aspect will not be discussed further here.

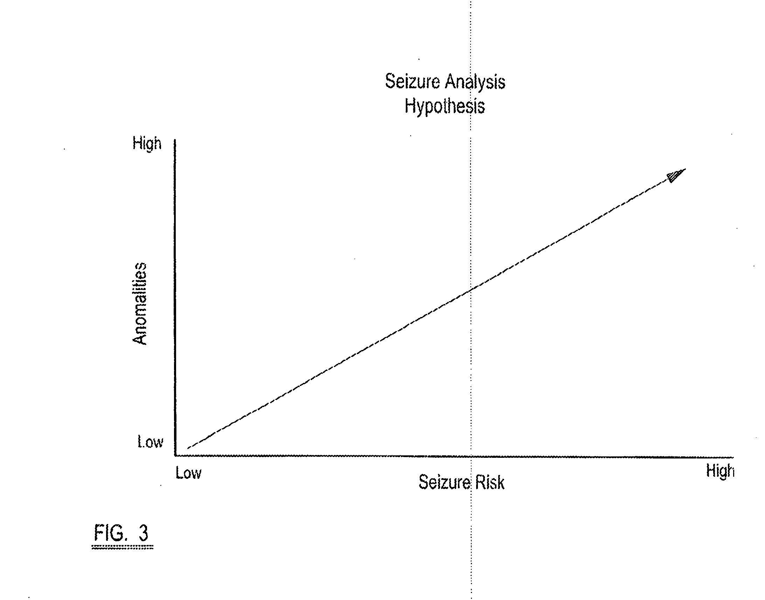

The electrical signals from the brain comprise rhythmic patterns and anomalies. By anomalies, we are referring to electrical signals which are random in nature and do not conform to rhythmic signal patterns. It is one premise of the invention that as the proportion of anomalies to rhythmic patterns in the electrical signal increases, then the likelihood of a neurological episode such as an epileptic fit also increases. This relationship is shown graphically at figure 3.

Specific identification of individual anomalies, such as signatures, is not necessary to provide useful information to predict or monitor the likelihood of a neurological event such as an epileptic seizure. Some specific anomalies are, however, indicators of the onset of a neurological event.

As well as detecting patterns using signal processing techniques and creating pattern ratios to identify threshold between patterns indicating a normal state and using those patterns to distinguish from a seizure state, one can also use more observational techniques to distinguish between the different classes of signal pattern.

The electrical signals received from EEGs are received as floating point data. The floating point data is then digitised and weighted in accordance with predetermined characteristics which can be pre-set or controlled by a user. Figure 11 shows such a weighted graph derived from the floating point data. In figure 11 the electronic readings are taken at a rate of 256 per second. The bolder line in figure 11 represents a floating point data which has been digitised and weighted, taken from the patient when in a normal state. The fainter trace represents electronic readings taken from the same patient preseizure and during seizure. Exactly the same scaling and weighting has been applied to the processed floating point data. It is clear from figure 11 that there is an almost rhythmic nature to the electronic reading when in the normal state. When in the seizure state, the electronic reading is clearly more erratic. An observation can be made looking at this data that the rhythmic electronic readings are characteristic of a normal state and the almost pseudorandom electronic readings are characteristic of a seizure state. These characterisations can be used through electronic processing/signal processing to determine a likelihood of the patient being in the normal state or in the seizure state. Usefully, when the electronic reading characteristics decay from the almost rhythmic pattern, observance of this decay can be used as a trigger to provide an alert that the patient is moving from a normal state towards a seizure state.

Embodiments of the invention use a number of different measures to make threshold decisions and some of those measures are discussed below. The invention bases decisions on pattern-derived parameters which may involve thresholding or reacting to a profile of a particular pattern-derived parameter. Thus, if a pattern derived parameter exceeds or falls below a predetermined or learned threshold, then a decision can be taken in response to that and an indicator given. Similarly, pattern-derived parameters can be profiled so when a parameter follows a particular trend such as decaying, then a decision can be taken in response to that and an indicator given. A pattern-derived parameter is a parameter derived from an observation of or operation on a digital data string which gives information about one or more patterns that recur in the digital data string. Examples of pattern-derived parameters are: the number of patterns identified in a data run; the proportion of patterns of a certain length compared to the total data payload; and combinations of these and including profiles or signatures of pattern-derived parameters such as monitoring the rate of change of a particular pattern-derived parameter.

The thresholds or profiles of pattern-derived parameters can be learnt by the monitoring or predicting system and varied according to individual characteristics of the user being monitored. Monitor learning uses known heuristics, neural network and artificial intelligence techniques.

A basic embodiment of a signal gathering and analysing system takes a neuronal activity signal either in digital form or converts it from analog to digital and then presents the signal as a character string. The character string may be in binary, hexadecimal or other base. The character string is preferably of the characters 0...9; A...F making up the hexadecimal character set. What is important is that the characters can provide a pattern of characters.

A sliding window of predetermined bit length or nibble length is placed over the data string and the data characters sitting within the window are considered to be a pattern. The pattern and the number of further occurrences of that pattern are logged as the window is slid over the entire data string. The window may be stepped incrementally through the data string bit by bit, in steps of multiple bits or potentially even pseudo-randomly. In basic terms, the system counts the number of occurrences of each pattern and creates various parameters or characterisations of the data based on pattern count. Variations in pattern count have been shown to provide an indication of whether or not the brain is in a pre-ictal or ictal period. The system also includes an output giving an indication of the onset of an ictal state based on the parameters derived from or characterisation of the pattern count.

The most basic embodiment of a monitoring or predicting system makes use of this relationship between pattern count and changes in neuronal activity to provide a monitoring or predicting system to provide a warning to a user based on an analysis which determines whether there has been a change in pattern count indicative of a change in neuronal activity indicative of onset of a seizure or the like. The analysis is based on internally stored historical ratios of pattern counts or can be processed by the monitoring or predicting device on the fly and compared with predetermined thresholds given the different parameters for the incoming data and the user.

The output of the monitoring or predicting system can be a wired output, a wireless output, a Bluetooth^{™} output, an optical output, an audio output or any other mechanism of alerting a user or reporting to a user. A particularly preferred method is the use of a traffic light indicator giving an alert status continually. The status of the indicator goes from green where there is no indication of onset of an ictal period, through amber where there is a potential risk of onset of an ictal period; to red where an ictal period is indicated as being imminent or ongoing.

The monitoring or predicting device is configured as a piece of electronic hardware with input connections to one or more neuronal activity sensors such as EEG electrodes which form part of a skull cap or an array of electrodes positioned on and attached to the skull. The device is preferably located on headgear or attached to the skull so that the path or distance from the or each sensor to the monitoring or predicting device is as short as possible. The device preferably has an internal power source but can be connected to an external power source.

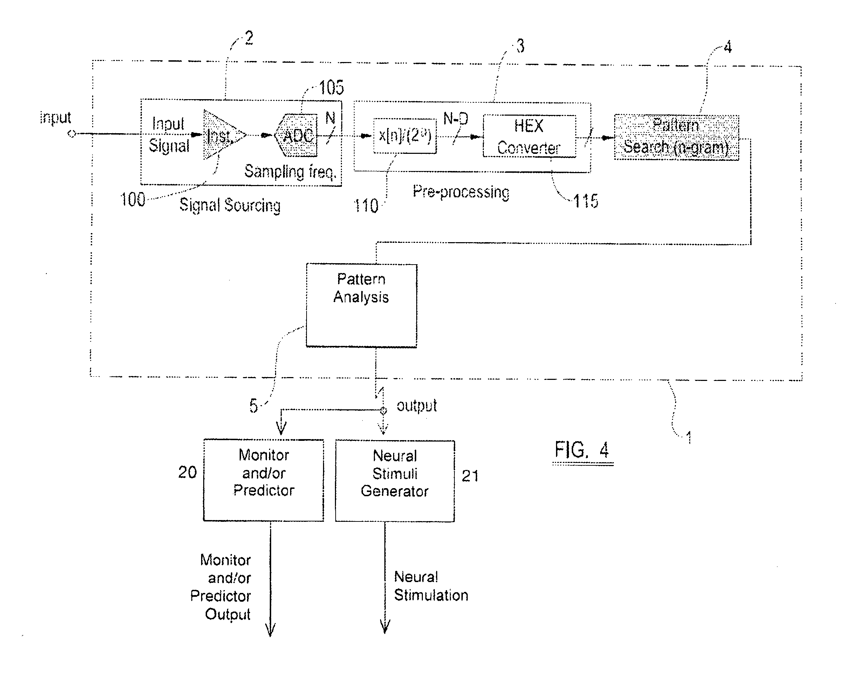

The monitoring or predicting device 1 in one embodiment of the invention shown in figure 4 comprises a number of modules defined by their functionality. In various embodiments, the modules are: either all held in a common housing of the monitoring or predicting device; or some modules are remote from the skull or body-located monitoring or predicting device and connected thereto by a wired or wireless connection.

There are four basic modules making up the monitoring or predicting device 1: a signal sourcing module 2 which receives input signals representing neuronal activity from sensors; a pre-processing module 3 which takes a sampled signal and creates a data string; a pattern search module 4 which analyses the data string and shows repeated patterns; and a pattern monitor module 5 which analyses the patterns and generates a monitor and/or predictor output in dependence on the analysed patterns.

Figure 14 shows a case list of available historical EEG data taken from patients in various conditions, usually either normal or abnormal, abnormal indicating pre-ictal or ictal state.

In a further embodiment of the device, as shown in figure 4, a neural stimulator is provided to furnish electrical or other stimuli to a part or parts of the brain. The stimuli are preferably furnished in response to the monitor and/or predictor output of the device.

Figure 15 shows the raw data and the process data for case 7. The raw data comprises the original floating point data from the EEG before it has been digitised and weighted. The process data shows the hexadecimal characters representing the digitised and weighted data from which patterns can be derived.

Figure 16 shows the patterns identified in case 7 in run 13 for which the data was captured during seizure. The size of the file is 40732 bits and for a 10 nibble pattern 4156 patterns were identified leaving 36576 anomalies giving an anomaly density or ratio of 89.8%.

Figure 17 shows the results for run 14 of case 7 which is data captured when the same patient in case 7 was in a normal state. Again, the file size is 40732 bits but the number of patterns identified is 39090 leaving only 1642 anomalies, giving an anomaly density or ratio of 4.03%. This conveys an immediate distinction between the pattern/anomaly density or ratio allowing immediate characterisation of the data signals as being either captured during a normal state or during a seizure state. The percentage of anomalies present during a seizure state is vastly greater than the percentage of anomalies present during a normal state. A threshold can be determined or even learned by the monitoring or predicting device which can constantly monitor, for example 10 second readings in real time and make a judgement on whether the pattern ratio or pattern threshold has been decayed or passed and provide an alert or prediction in response to monitoring of this pattern-derived parameter. Conventional pattern analysis and pattern derivation mechanisms can be used to derive, identify, count and monitor patterns.

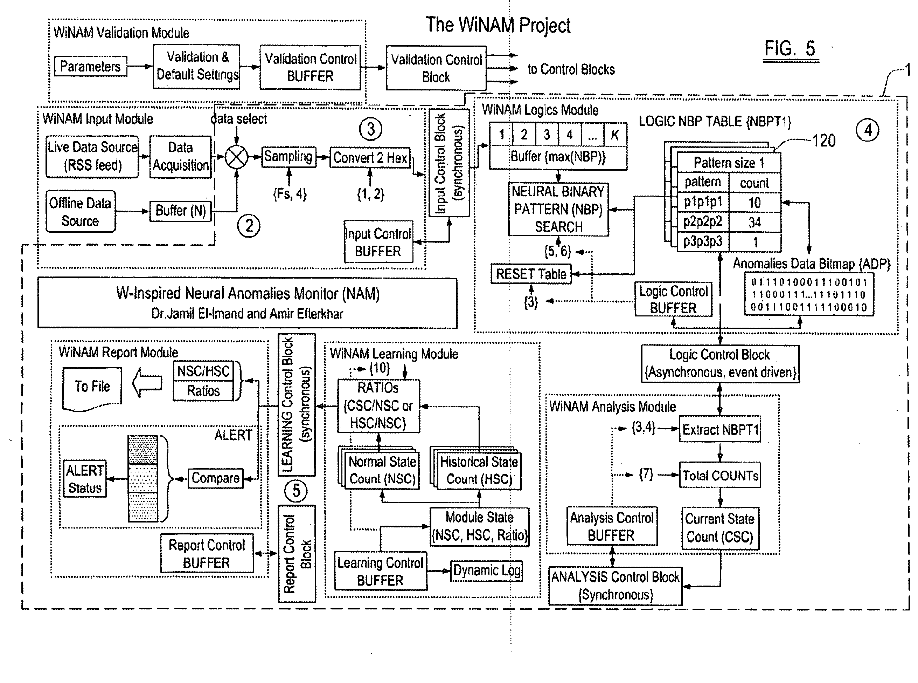

Figure 5 shows the modules 2,3,4 and 5 of a monitoring or predicting device 1 as part of a larger and more detailed network which includes the facility to stream live data or run stored data through the modules.

Referring to figure 4, the signal sourcing module 2 has an amplifier 100 or pre-amplifier to receive neuronal activity input signals (an analog signal) preferably from EEG electrodes. Downstream of the amplifier there are one or more analog to digital converters 105 (or a multiplexed analog to digital converter) operating at a sampling frequency fs and having as their input the respective amplified EEG signals from the electrodes 10.

The sampled output of the analog to digital converters 110 is a binary string which is preferably converted to hexadecimal by HEX converter 115. The use of hexadecimal is particularly helpful to gain a visible and direct appreciation of the presence of patterns in the signal being monitored.

An analog-to-digital converter is used with typical sampling frequencies (fs) of 128-512Hz for EEG and ECoG to 10-30KHz for single neuron and local field potential (LFP) signals. The conversion, depending upon the application can result in 8-16bit data. When stored for software (and microcontroller hardware) this information is represented at its lowest level in binary, but in a higher level of abstraction in hexadecimal (HEX). Hence, the data is already available in an alphanumeric format.

The hexagonal output is fed to the pattern search module 4 which is configured in this example as an n-gram model.

Additionally, we can adjust the level of lossiness of the data representation by dividing the data (an N bit number) by 2^{D} where D is an integer, to result in a reduced data format - i.e. reducing a 16bit number to an 8bit one by dividing down with D = 8.

The n-gram process in the pattern search module 4 extracts any patterns in the signals. Once patterns are extracted the number of significant patterns are counted. A significant pattern is a pattern that has occurred more than 2 times but other threshold limits can be selected and may be usefully varied for different pattern sizes. The greater the pattern size, i.e. string length, the less repeating patterns there will be.

The pattern count is monitored and when the pattern count drops below a historically derived threshold stored in the pattern monitor, the pattern monitor outputs a change of status. A significant pattern count is quantified in two ways: (1) to count out of the number of significant patterns the total number of occurrences of all these patterns and (2) out of the patterns found what percentage were significant. The former is shown in the below results, the latter method quantified similar results so is not shown here. These pattern counts can then be quantified as a ratio between a current window of analysis and a previous window during an inter-ictal state (ictal refers to the state during a seizure).

The hexadecimal output is sampled and patterns identified and counted.

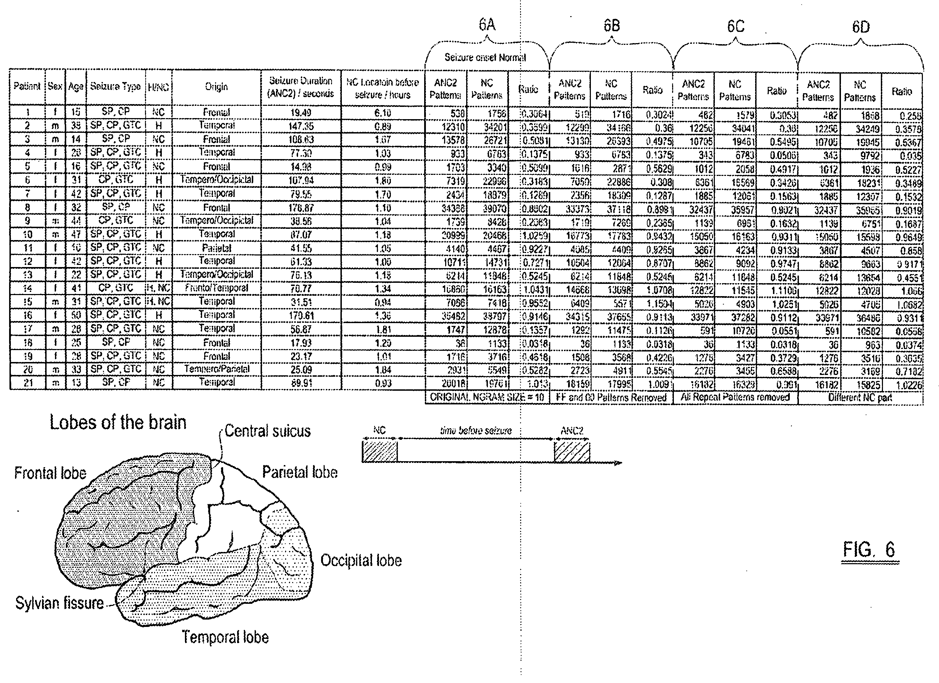

In figure 6, there are four sets of results 6A,6B,6C and 6D. The "NC" columns are data taken in the time prior to a neurological event (pre-ictal). The "ANC2" columns are data taken during a seizure onset and during the event (ictal) - see also the timing diagram at the foot of the table in figure 6.

6A gives the raw results. 6B recognises that certain patterns occur very frequently particularly those patterns representing a saturated signal for a null signal which in hexadecimal terms would equate to "00" or "FF". These patterns are therefore excluded from the list of patterns. 6C removes all repeat patterns from the list of patterns. A repeat pattern is a sub-set of a pattern which occurred in a larger pattern size pattern list.

The other figures give similar pattern matrices for 9, 8, 7 and 6 nibbles taken for the same data string. Tables I to V show the first page of patterns and frequency of occurrence for the five pattern sizes of 10 to 6 nibbles.

Preferably the data is sampled as 6, 7, 8, 9 or 10 nibbles from a sliding window applied to the hexadecimal data output string and the occurrence of each individual distinct nibble pattern is logged. In the 10 nibble pattern matrix shown in Table I, the two most popular occurring 10 nibble patterns in the "NC" data acquisition period are 020100FFFE and 20100FFFEF which patterns both occur 5 times in the "NC" data acquisition period. Many other 10 nibble patterns occur during the "NC" period.

The signal sourcing module receives input signals S1-S7 representing neuronal activity from one or more EEG sensors 10 (see figures 1 and 2) attached to the skull in a conventional manner (of both attachment and/or array). The input signals in this example are electrical signals S1-S7 direct from EEG sensors 10. In other embodiments, the input signals may be remotely streamed from a live feed or a recorded data set.

In the preferred embodiment, the number of repeated patterns in NC is compared to the number of repeated patterns in ANC2. There are usually more repeated patterns in NC rather than ANC2 during the actual seizure. As a consequence, there are less patterns identifiable during a seizure, meaning that there are also more anomalies occurring during seizure hence the premise of the invention that an increase in the proportion of anomalies to repeated patterns is an indicator or predictor of the onset of a neurological event such as an epileptic seizure.

A relative increase in the number of anomalies is a direct indicator of the onset of a neurological episode and is useful information to allow the device to perform an episode prediction function. The likelihood of the onset of a neurological episode increases as the number of anomalies increases.

Aspects of the invention deal with one of the bottlenecks of analysis of epileptic seizure activity. An aspect of the invention allows the ability to work with consistent data which is well annotated and databased to establish a framework for future work and storage of results into the same framework. The system shown at figure 5 provides this framework.

Figure 5 shows data acquisition, user interface and processing blocks. In theory each of these components could be placed in a different technological implementation, such as the acquisition being an implantable neural monitoring or predicting device, the user interface being on a mobile phone or PC and the processing units being a web-accessed cloud (such as the Amazon Elastic Compute Cloud). The distribution of these elements will vary depending upon the signal processing requirements (computational complexity) and application space.

Pattern analysis of historical data yields sets of parameters concerning the patterns. Predictions or decisions on whether a neural event is upcoming can be taken by comparing in either relative or absolute terms real-time patterns with stored parameters, pre-determined patterns and thresholds. The monitoring or predicting device provides an output indicative of whether a neural event is unlikely, likely or imminent, much like a traffic light output: red, amber and green.

The electrodes or electrical contacts that are used to detect neuronal activity from the brain are the inputs to the monitoring or predicting device. These inputs may be reversed to provide a stimulus output. The present invention also includes the provision of neuronal stimulation to a part or parts of the brain.

The stimulus may be provided in response to any of the parameters measured or monitored by the monitoring or predicting device, such as a change in pattern count indicative of a change in neuronal activity, an onset, a seizure or the like. Thus, the traffic light output from the device can be used to trigger a neural stimulation, perhaps targeted stimulation, in an attempt to ameliorate, offset, delay or avoid entirely a neurological episode such as an epileptic seizure. The neural stimulation is provided by a neural stimuli generator 21 which can be a part of the device or connectable to a device output, potentially wirelessly, or wired.

Referring to figure 6, this data was obtained from the University Hospital of Freiburg Epilepsy Centre, Germany. The data used were pre-sampled at 256Hz and quantised using a 128 channel 16-bit data acquisition. All 21 patients' first seizure was used from this data set. There is a pre-ictal period of up to 1 hour in most cases with seizure durations varying from 15-170 seconds. There were multiple types of seizures present including: simple partial, complex and general tonic-clonic.

Conventional pattern analysis techniques were used. The invention does not relate to the analysis technique but in the identification that patterns and pattern ratios of the digitised and sampled data are characteristic of a patient being in normal, pre-ictal and seizures states.

Two sets of tests were conducted with this data. The first set aimed to quantify whether there were pattern differences between seizure/ictal areas when compared against inter and pre-ictal periods. To do this, sample pre-ictal periods equal in size to the seizure period were extracted. The average pattern count between each of 10 sections of pre-ictal data is computed in order to compare against the seizure pattern count. For this analysis we used D = 8 and analysed the data for n-gram sizes of 12 and 14, where one token was one electrical reading in 1 byte or 2HEX characters. These results (shown in Table VI) show that in most cases the pattern count (P) compared to the seizure count (S) was considerably different; 18 out of 21 cases showed a ratio that indicated a greater than 25% change in pattern count. These results aim to distinguish pre-ictal from ictal periods.

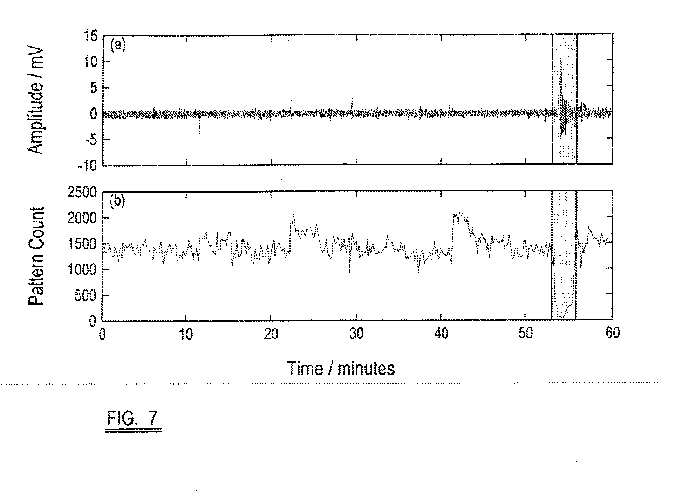

The second set of tests analysis used the full pre-ictal period, segmented into 5 and 10 second windows. We analysed the data using D = 8 and with n-gram sizes 10 and 14. A typical pattern count found in these data sets is shown in figure 7. The results for all 21 patients are shown in Table VII. Table VII shows heuristic results (related to pattern count changes) when analysing the full data sets. The descriptions refer to the changes that occur that are visible changes compared to the pre-ictal period.

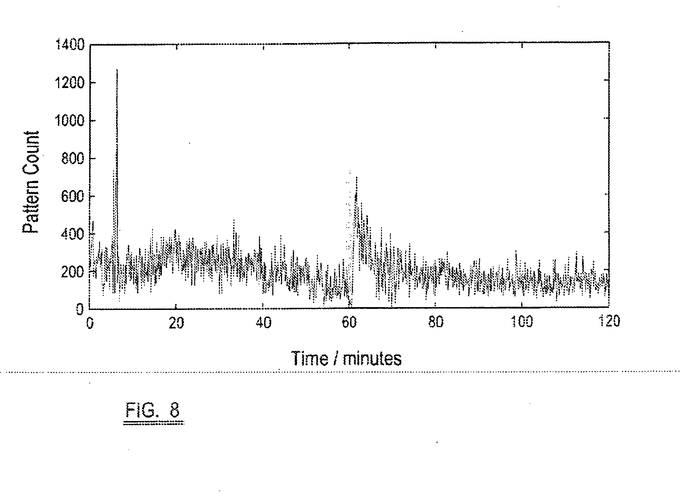

It is clear patterns exist and interestingly some patterns change or exist for certain n-gram sizes but not for others. An interesting pattern was an increase followed by a slow decrease over several minutes - see sharp increase at 60 minutes in figure 8. These findings conclude that out of the 21 patients, 18 can be detected using the features outlined in figure 6. Table VII shows patterns at different n-gram sizes. Patterns are identical using different n-gram sizes while making sure that patterns in an n-gram of 12 are not replicated in an n-gram size of 10, i.e. making sure that patterns are unique and not a subset of a larger pattern in the data. Also, one needs to identify data parameters (e.g. types of seizure) to identify if certain patterns correlate with patient-specific information.

To gather further historical data and develop pattern parameters and thresholds there is disclosed a modular analysis framework as shown in figure 5.

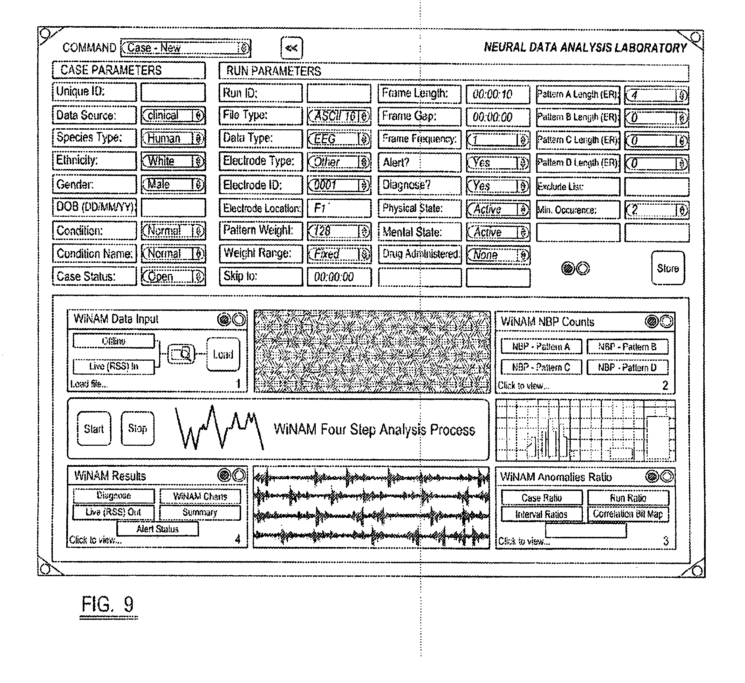

The system of figure 5 is an open online and/or real-time analysis tool (www.winam.net) with an SQL database that is used to examine multiple cases and runs of data sets and presents in a webpage form as shown I figure 9. As in figure 5 the structure is such that the data sourcing can be through an RSS feed, or offline data source. The processing (n-gram) is implemented through a separate processing cluster allowing multiple parallel processing efficiently. This is an open system that allows users to freely access and carry out the algorithmic techniques described in this paper. The database structure itself is designed to allow users to input multiple patient cases, and for each case run particular data sets (EEG, ECoG, ECG etc...) or parts of these data sets.

Figure 18 shows a second graphical user interface designed to analyse neuronal activity data signals in accordance with an embodiment of the invention. This interface is used to set the parameters, in real-time or offline, of the logics module 4 and/or the downstream analysis module.

In Figure 18, a number of parameters may be monitored and/or adjusted and applied to the data in real time (and not just post-analysis). The listed parameters are not exclusive and other parameters or sub-parameters can also be modified.

The "weighting" parameter refers to the rounding of the EEG signal. For example, the EEG signal may be sampled at 5Hz, and then the number of samples may be divided by 128 to remove noise - effectively "zooming in" on the results. The signal then needs to be rounded as a 5Hz sample frequency divided by 128 will not give an integer number of samples. In a preferred embodiment, the signal is rounded up.

The "interval" parameter is the window length the user wishes to process cycles in. In one embodiment, the interval length may be 1 minute.

The "frequency" parameter is a frame frequency and relates to the number of frames to be processed. If, for example, the user has 1 hour of data and the 11th to 20th minutes are of interest, the user can skip to the 11th minute and select how many frames will be read - e.g. 20 frames at a 30s interval length.

The "optimiser" parameter determines the optimum pattern length to determine a suitable anomaly ratio. For example, with a pattern length of 2 bits it is likely there will be very few or no anomalies, but with a pattern length of 20 bits, it is likely there will be several anomalies. The optimiser effectively sets a benchmark for the anomaly ratio. In a preferred embodiment, the optimiser parameter determines the pattern length of each pattern type A, B, C, D, (see, for example, Figure 17, where the length of pattern A is 10 nibbles, and patterns B,C and D are 0) such that anomaly ratio is less than 10%. The optimiser can automatically determine the ideal settings for each of the pattern groups (shown as GNBP(A-D) on the user interface). The pattern group settings may be overridden manually by adjusting, respectively, GNBP(A-D) individually or jointly.

The "SD" parameter control relates to the threshold standard deviation of the results.

Other importing functionality is readily implemented as is reporting documentation to further visualise, analyse the results and further develop pattern-based parameters on which to base neuronal event monitoring or predicting decisions.

When used in this specification and claims, the terms "comprises" and "comprising" and variations thereof mean that the specified features, steps or integers are included. The terms are not to be interpreted to exclude the presence of other features, steps or components.

The features disclosed in the foregoing description, or the following claims, or the accompanying drawings, expressed in their specific forms or in terms of a means for performing the disclosed function, or a method or process for attaining the disclosed result, as appropriate, may, separately, or in any combination of such features, be utilised for realising the invention in diverse forms thereof.

### Annex:

### WiNam

### Core Logic

### Jamil El-Imad

### May 2010

### Neural Binary Pattern Storage Table

### (NBPST)

*Each pattern group from(A, B, C or D) has a length defined by Pattern_n_length (where n = a, b, c or d) will have a storage table where'unique' patterns are stored. Each pattern stored has an associated count and the last offset (from the start of frame*).

| **Pattern A -** | (validation A>B>C>D) |
|---|---|
| Pattern_a_name: | xxxxxxxx (Key) |
| Pattern_a_count: | 32,768 (16bit / 2byte) |
| Pattern_a_last_offset: | 32,768 (16bit / 2byte) |

### Sample:

| | | | | |
|---|---|---|---|---|
| Pattern_A_name | A1 10 7F 33 | 3F 51 A2 9F | .... | 75 00 D1 01 |
| Pattern_A_count | 1 | 3 | .... | 1 |
| Pattern_A_last_offset | 1 | 11 | .... | 23 |

| **Pattern B** | | |
|---|---|---|
| Pattern_b_name: | xxxxxxxx (Key) | |
| Pattern_b_count: | 32,768 (16bit / 2byte) | |
| Pattern_b_last_offset: | 32,768 (16bit / 2byte) | |

| **Pattern C** | | |
|---|---|---|
| Pattern_c_name: | XXXXX (Key) | |
| Pattern_c_count: | 32,768 (16bit / 2byte) | |
| Pattern_c_last_offset: | 32,768 (16bit / 2byte) | |

| **Pattern D** | | |
|---|---|---|
| Pattern_d_name: | XXXXXX (Key) | |
| Pattern_d_count: | 32,768 (16bit / 2byte) | |
| Pattern_d_last_offset: | 32,768 (16bit / 2byte) | |
| Pattern_a_length: | *As per user input*: | 2-24 byte patterns |
| Pattern_b_length: | | 2-24 byte patterns |
| Pattern_c_length: | | 2-24 byte patterns |
| Pattern_D_length: | | 2-12 byte patterns |

*Each data value is 1 byte in length (8bits). Pattern_N_length determines the number of 1 byte patterns that constitute a pattern - example: Pattern_A_length = 3 refers to 'XX XX XX', such as '10 7F 33'.*

| | |
|---|---|
| Offset: | 32,768 (16bit / 2byte), initial value -1 |
| LLA: | 32,768 (16bit / 2byte), initial value o |

### NBP Process

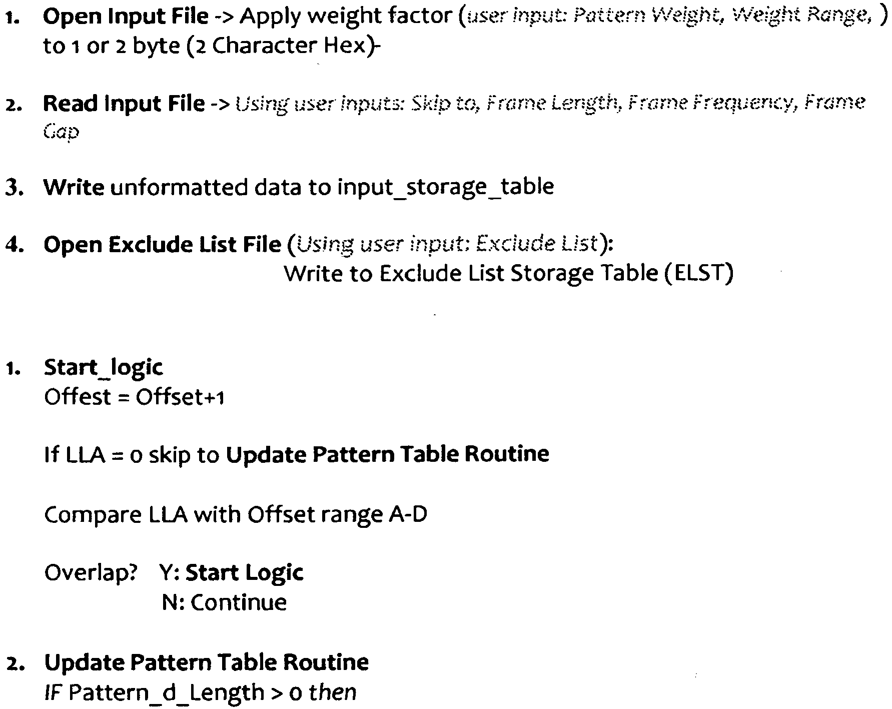

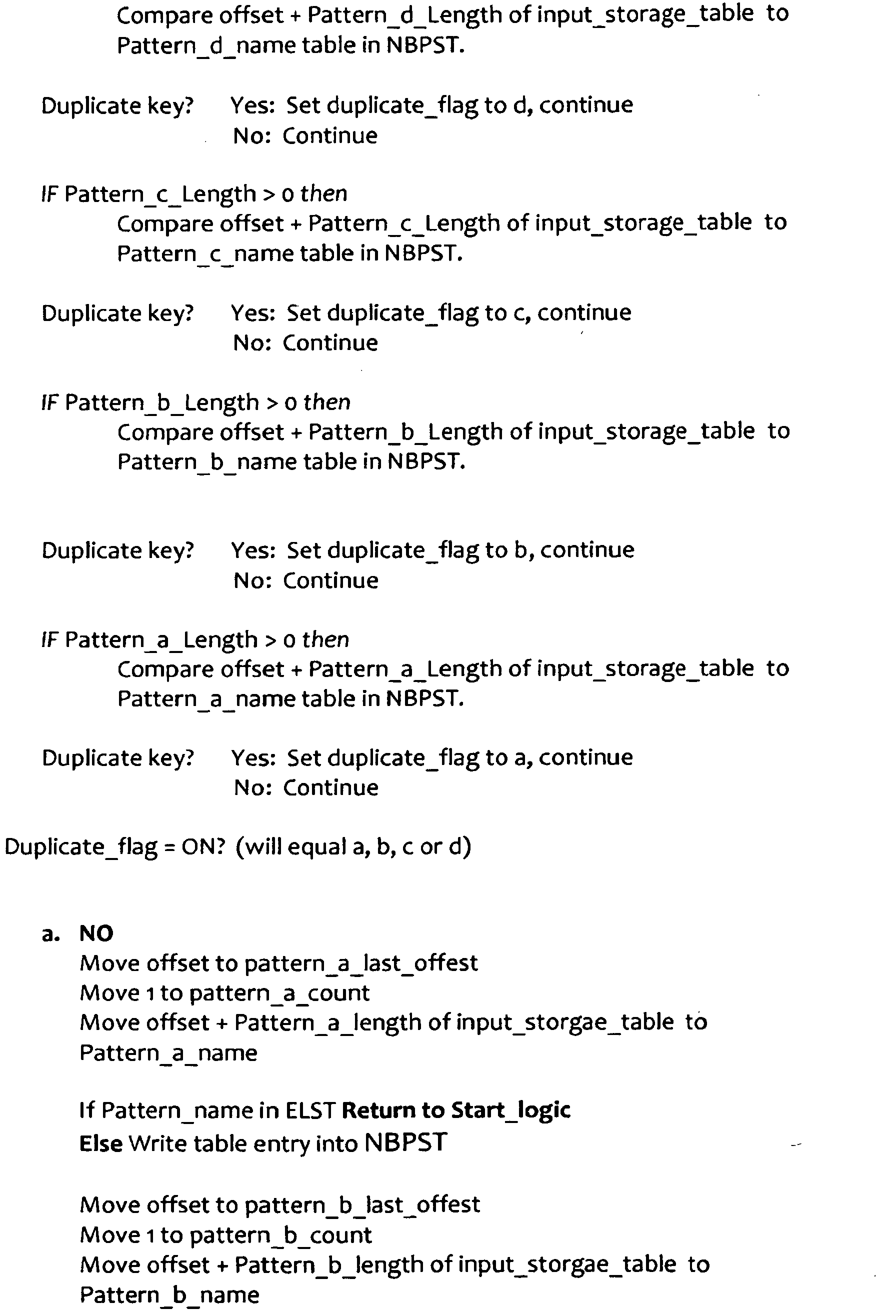

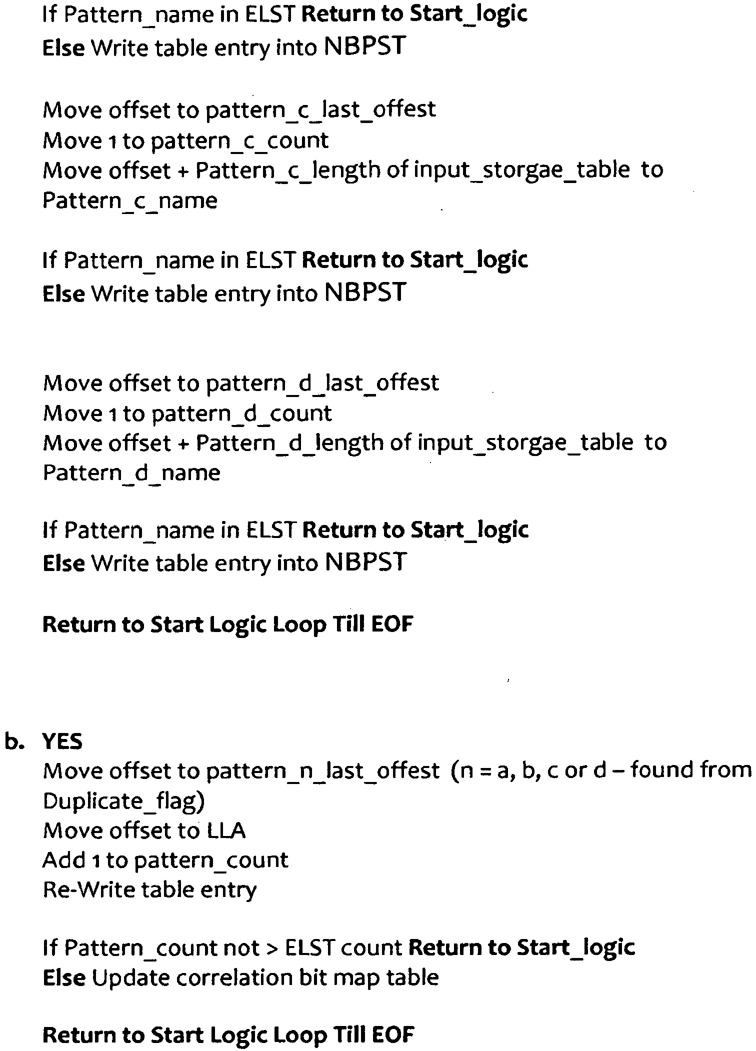

## Claims

1. A monitoring or predicting system (1) to detect the onset of a neurological episode, the system comprising:
a neurological electrical input (2), the input being a digital representation of a neurologically derived signal;
a converter (3) adapted to convert the digital signal into a digital data string;
a pattern analyser (4) adapted to identify recurring patterns in the digital data string; and
a monitor (5) adapted to measure a pattern-derived parameter related to one or more of:
a count of said recurring patterns in the digital data string;
a proportion of said recurring patterns in the digital data string;
a rate of change of a count of said recurring patterns in the digital data string; and
a rate of change of a proportion of said recurring patterns in the digital data string,
wherein an output from the monitor (5) gives an indication of the onset or occasion of a neuronal activity in dependence on the pattern-derived parameter.

2. The system of claim 1, wherein the digital data string is a character data string, a binary data string or a hexadecimal data string.

3. The system of any preceding claim, wherein the system further comprises a neural stimuli generator for stimulating a part of a brain.

4. The system of any preceding claim, wherein the neurological electrical input (2) is provided by electrodes (10) and is located on headgear; and
the other modules of the system are all located on the headgear; or
one or more of the other modules is remote from the headgear and connected thereto by a wired or wireless connection.

5. The system of any preceding claim, wherein the output of the monitoring or predicting system is a wired output, a wireless output, a Bluetooth output, an optical output, or an audio output.

6. A method of detecting the onset of a neurological episode comprising:
receiving a neurological electrical input comprising a digital representation of a neurologically derived signal;
converting the digital signal into a digital data string;
identifying recurring patterns in the digital data string;
monitoring a pattern-derived parameter related to one or more of:
a count of said recurring patterns in the digital data string;
a proportion of said recurring patterns in the digital data string;
a rate of change of a count of said recurring patterns in the digital data string; and
a rate of change of a proportion of said recurring patterns in the digital data string; and
providing an output giving an indication of the onset or occasion of a neuronal activity in dependence on the pattern-derived parameter.

7. The method of claim 6, further comprising:
weighting the digital signal when converting the digital signal into a digital data string.

8. The method of claim 6 or 7, further comprising:
sampling the digital data string with a bit length of 6, 7, 8, 9 or 10 bits.

9. The method of any of claims 6 to 8, further comprising:
reacting to a profile of a particular pattern-derived parameter.

10. The method of any of claims 6 to 9, further comprising:
counting significant recurring patterns.

11. The method of any of claims 6 to 10, further comprising:
excluding patterns in the data string that are identified as null signals from the significant recurring pattern count.

12. The method of any of claims 6 to 11, further comprising:
detecting or identifying the type of neurological episode.

13. The method of any of claims 6 to 12, wherein the output giving an indication of the onset or occasion of a neuronal activity is determined based on either:
analysing internally stored historical ratios of pattern counts; or
processing by a monitoring device and comparing with a predetermined threshold.

14. The method of claim 13, wherein the predetermined threshold is learned from the user profile using known heuristics, neural network and/or artificial intelligence techniques, or determined by the total number of significant patterns and/or a percentage of significant patterns found.

15. The method of any of claims 6 to 14, further comprising:
plotting the digital data on a graph.

## Patentansprüche

1. Überwachungs- oder Vorhersagesystem (1) zur Erfassung des Einsetzens eines neurologischen Schubs, wobei das System umfasst:
eine neurologische elektrische Eingabe (2), wobei die Eingabe eine digitale Darstellung eines neurologisch abgeleiteten Signals ist;
einen Wandler (3), der dazu ausgelegt ist, das digitale Signal in eine digitale Datenreihe umzuwandeln;
einen Musteranalysator (4), der dazu ausgelegt ist, wiederkehrende Muster in der digitalen Datenreihe zu identifizieren; und
einen Monitor (5), der dazu ausgelegt ist, einen musterabgeleiteten Parameter zu messen, der sich auf eine oder mehrere der folgenden bezieht:
eine Zählung der wiederkehrenden Muster in der digitalen Datenreihe;
ein Verhältnis der wiederkehrenden Muster in der digitalen Datenreihe;
eine Änderungsrate einer Zählung der wiederkehrenden Muster in der digitalen Datenreihe; und
eine Änderungsrate eines Verhältnisses der wiederkehrenden Muster in der digitalen Datenreihe,
wobei eine Ausgabe von dem Monitor (5) eine Anzeige des Einsetzens oder des Auftretens einer neuronalen Aktivität in Abhängigkeit von dem musterabgeleiteten Parameter angibt.

2. System nach Anspruch 1, wobei die digitale Datenreihe eine Zeichendatenreihe, eine binäre Datenreihe oder eine hexadezimale Datenreihe ist.

3. System nach irgendeinem vorhergehenden Anspruch, wobei das System ferner einen Neuralreizgenerator zum Stimulieren eines Teils eines Gehirns umfasst.

4. System nach irgendeinem vorhergehenden Anspruch, wobei die neurologische elektrische Eingabe (2) von Elektroden (10) bereitgestellt wird und sich auf einer Kopfbedeckung befindet; und
sich die anderen Module des Systems alle auf der Kopfbedeckung befinden; oder
eines oder mehrere der anderen Module von der Kopfbedeckung entfernt und mit dieser durch eine drahtgebundene oder drahtlose Verbindung verbunden ist.

5. System nach irgendeinem vorhergehenden Anspruch, wobei die Ausgabe des Überwachungs- oder Vorhersagesystems eine drahtgebundene Ausgabe, eine drahtlose Ausgabe, eine Bluetooth-Ausgabe, eine optische Ausgabe oder eine Audioausgabe ist.

6. Verfahren zur Erfassung des Einsetzens eines neurologischen Schubs, umfassend:
Empfangen einer neurologischen elektrischen Eingabe, die eine digitale Darstellung eines neurologisch abgeleiteten Signals umfasst;
Umwandeln des digitalen Signals in eine digitale Datenreihe;
Identifizieren wiederkehrender Muster in der digitalen Datenreihe;
Überwachen eines musterabgeleiteten Parameters, der sich auf eine oder mehrere der folgenden bezieht:
eine Zählung der wiederkehrenden Muster in der digitalen Datenreihe;
ein Verhältnis der wiederkehrenden Muster in der digitalen Datenreihe;
eine Änderungsrate einer Zählung der wiederkehrenden Muster in der digitalen Datenreihe; und
eine Änderungsrate eines Verhältnisses der wiederkehrenden Muster in der digitalen Datenreihe; und
Bereitstellen einer Ausgabe, die eine Anzeige des Einsetzens oder des Auftretens einer neuronalen Aktivität in Abhängigkeit von dem musterabgeleiteten Parameter angibt.

7. Verfahren nach Anspruch 6, ferner umfassend:
Gewichten des digitalen Signals, wenn das digitale Signal in eine digitale Datenreihe umgewandelt wird.

8. Verfahren nach Anspruch 6 oder 7, ferner umfassend:
Abtasten der digitalen Datenreihe mit einer Bitlänge von 6, 7, 8, 9 oder 10 Bits.

9. Verfahren nach irgendeinem der Ansprüche 6 bis 8, ferner umfassend:
Reagieren auf ein Profil eines bestimmten musterabgeleiteten Parameters.

10. Verfahren nach irgendeinem der Ansprüche 6 bis 9, ferner umfassend:
Zählen signifikanter wiederkehrender Muster.

11. Verfahren nach irgendeinem der Ansprüche 6 bis 10, ferner umfassend:
Ausschließen von Mustern in der Datenreihe, die aus der Zählung signifikanter wiederkehrender Muster als Nullsignale identifiziert sind.

12. Verfahren nach irgendeinem der Ansprüche 6 bis 11, ferner umfassend:
Erfassen oder Identifizieren des Typs des neurologischen Schubs.

13. Verfahren nach irgendeinem der Ansprüche 6 bis 12, wobei die Ausgabe eine Anzeige des Einsetzens oder Auftretens einer neuronalen Aktivität bestimmt wird auf der Basis von entweder:
einer Analyse intern gespeicherter Verlaufsverhältnisse von Musterzählungen; oder
einer Verarbeitung durch eine Überwachungsvorrichtung und eines Vergleichs mit einem vorgegebenen Schwellwert.

14. Verfahren nach Anspruch 13, wobei der vorgegebene Schwellwert aus dem Benutzerprofil unter Verwendung bekannter Heuristikverfahren, Neuralnetz- und/oder Künstliche-Intelligenz-Techniken erfahren wird oder durch die Gesamtzahl signifikanter Muster und/oder eines Prozentsatzes signifikanter Muster, die gefunden werden, bestimmt wird.

15. Verfahren nach irgendeinem der Ansprüche 6 bis 14, ferner umfassend:
Eintragen der digitalen Daten in einer grafischen Darstellung.

## Revendications

1. Système de surveillance ou de prédiction (1) destiné à détecter le début d'un épisode neurologique, le système comprenant :
une entrée électrique neurologique (2), l'entrée étant une représentation numérique d'un signal neurologiquement dérivé ;
un convertisseur (3) adapté pour convertir le signal numérique en une chaîne de données numériques ;
un analyseur de profil (4) adapté pour identifier des profils récurrents dans la chaîne de données numériques ; et
un moniteur (5) adapté pour mesurer un paramètre dérivé d'un profil, relatif à un ou plusieurs des éléments suivants :
un décompte desdits profils récurrents dans la chaîne de données numériques ; une proportion desdits profils récurrents dans la chaîne de données numériques ;
un taux de variation d'un décompte desdits profils récurrents dans la chaîne de données numériques ; et
un taux de variation d'une proportion desdits profils récurrents dans la chaîne de données numériques, dans lequel une sortie du moniteur (5) donne une indication du début ou de l'occasion d'une activité neuronale en fonction du paramètre dérivé du profil.

2. Système selon la revendication 1, dans lequel la chaîne de données numériques est une chaîne de données de caractères, une chaîne de données binaires ou une chaîne de données hexadécimales.

3. Système selon l'une quelconque des revendications précédentes, dans lequel le système comprend en outre un générateur de stimuli destiné à stimuler une partie d'un cerveau.

4. Système selon l'une quelconque des revendications précédentes, dans lequel l'entrée électrique neurologique (2) est fournie par des électrodes (10) et est situé sur un casque ;
et les autres modules du système sont tous situés sur le casque ;
ou un ou plusieurs des autres modules sont déportés du casque et y sont connectés par une connexion câblée ou sans fil.

5. Système selon l'une quelconque des revendications précédentes, dans lequel la sortie du système de surveillance ou de prédiction est une sortie câblée, une sortie sans fil, une sortie Bluetooth, une sortie optique ou une sortie audio.

6. Procédé de détection du début d'un épisode neurologique, comprenant les étapes consistant à :
recevoir une entrée électrique neurologique comprenant une représentation numérique d'un signal neurologiquement dérivé ;
convertir le signal numérique en une chaîne de données numériques ;
identifier les profils récurrents dans la chaîne de données numériques ;
surveiller un paramètre dérivé d'un profil, relatif à un ou plusieurs des éléments suivants :
un décompte desdits profils récurrents dans la chaîne de données numériques ;
une proportion desdits profils récurrents dans la chaîne de données numériques ;
un taux de variation d'un décompte desdits profils récurrents dans la chaîne de données numériques ; et
un taux de variation d'une proportion desdits profils récurrents dans la chaîne de données numériques ; et
fournir une sortie donnant une indication du début ou de l'occasion d'une activité neuronale en fonction du paramètre dérivé du profil.

7. Procédé selon la revendication 6, comprenant en outre l'étape consistant à :
pondérer le signal numérique lors de la conversion du signal numérique en une chaîne de données numériques.

8. Procédé selon la revendication 6 ou 7, comprenant en outre l'étape consistant à :
échantillonner la chaîne de données numériques avec une longueur de bits de 6, 7, 8, 9 ou 10 bits.

9. Procédé selon l'une quelconque des revendications 6 à 8, comprenant en outre l'étape consistant à :
réagir à un profil d'un paramètre particulier dérivé du profil.

10. Procédé selon l'une quelconque des revendications 6 à 9, comprenant en outre l'étape consistant à :
compter les profils récurrents significatifs.

11. Procédé selon l'une quelconque des revendications 6 à 10, comprenant en outre l'étape consistant à :
exclure du décompte de profils récurrents significatifs les profils de la chaîne de données qui sont identifiés comme des signaux nuls.

12. Procédé selon l'une quelconque des revendications 6 à 11, comprenant en outre l'étape consistant à :
détecter ou identifier le type d'épisode neurologique.

13. Procédé selon l'une quelconque des revendications 6 à 12, dans lequel la sortie donnant une indication du début ou de l'occasion d'une activité neuronale est déterminée sur la base :
de l'analyse des rapports historiques stockés en interne des décomptes de profils ; ou
du traitement par un dispositif de surveillance et de la comparaison à un seuil prédéterminé.

14. Procédé selon la revendication 13, dans lequel le seuil prédéterminé est appris à partir du profil de l'utilisateur au moyen d'heuristiques connues, d'un réseau neuronal et/ou de techniques d'intelligence artificielle, ou déterminé par le nombre total de profils significatifs et/ou par un pourcentage de profils significatifs trouvés.

15. Procédé selon l'une quelconque des revendications 6 à 14, comprenant en outre l'étape consistant à :
tracer les données numériques sur un graphique.
